# EUROPEAN PATENT APPLICATION

(11) **EP 4 636 430 A1**
(43) Date of publication of application: **22.10.2025**
(21) Application number: 24170190.3
(22) Date of filing: 15.04.2024
(51) Int. Cl.: G01R 33/565, A61B 5/055, A61B 5/00, G01R 33/3415

(54) **SYSTEM AND METHOD FOR MOTION DETECTION IN MRI USING PILOT TONE SIGNAL**

(71) Applicant: Siemens Healthineers AG, 91301 Forchheim (DE)
(72) Inventor: Hilbert, Tom, 1006 Lausanne (CH); Kober, Tobias, 1025 St-Sulpice (CH); Philippe, Jocelyn, 1004 Lausanne (CH); Splitthoff, Daniel Nicolas, 91080 Uttenreuth (DE)
(74) Representative: Palaci, Ismaël

(57) **Abstract**

The invention relates to a system and a method for automatically detecting, during an MRI session, a motion of a biological object (210) placed in an examination volume (221) of an MRI apparatus (220), the method (100) comprising:
- emitting (101), in said examination volume (221) and during said MRI session , a pilot tone signal - hereafter PT signal -, wherein the PT signal is emitted at a frequency F_pt close to the Larmor frequency, but outside a range of frequencies used by the MRI apparatus (220) for imaging said biological object (210);
- acquiring (102), by the MRI apparatus (220) the PT signal;
- detecting (103), by a motion detection algorithm, in real time and from the acquired PT signal, a motion of the biological object;
- automatically triggering (104) an action, during said MRI session, in function of the detected motion

## Description

### Technical Field

The present disclosure is directed, in general, to Magnetic Resonance Imaging (MRI) techniques used for imaging a biological object, like a brain. More specifically, the present invention is directed to methods and systems for detecting a motion of such biological object during imaging.

### Background Art

MRI has become an essential tool for today's clinical diagnostics; e.g., for diagnosis and follow-up of neurodegenerative diseases such as dementia and multiple sclerosis, and is widely used in clinical routine.

However, one remaining challenge of MR imaging concerns motion occurring during image acquisition that results in artefacts in the acquired image, potentially rendering the whole MRI exam unusable. Indeed, the imaged biological object is supposed to be absolutely still. This is especially problematic for patients in pain, elderly or very young patients. But even for compliant patients, holding still for several minutes up to 20-30 minutes of a regular clinical exam is challenging. Typical motion artifacts are image blurring or ringing. An MRI exam is typically not composed of a single image acquisition, but of several in order to obtain different clinically relevant image contrasts. Each of these image acquisitions in an examination can take several minutes. In a best-case scenario, the technician operating the MRI notices that the images were corrupted by motion and repeats the specific image acquisition immediately. But even this best case scenario has a significant impact: patients must stay longer in the scanner, the scanner schedule is affected, and total cost increases. In the worst case, motion artefacts are only detected during the radiologist's reading, which can require to call in the patient for another MRI exam, obviously straining both patient's comfort and healthcare costs. In addition, the delay might severely prolong the time until diagnosis for the patient. Also, in cognitive neurosciences, despite the higher degree of compliance by healthy volunteers, small motion during functional MRI runs can induce spurious, non-neurological signal contributions that can be confounded with brain activity.

Given the detrimental impact of motion, various methods to overcome motion susceptibility of MRI have been developed over the past decades. They can be roughly divided in prospective and retrospective motion correction methods [1]. Retrospective motion correction techniques attempt to remove motion artifacts after the data for image reconstruction was acquired, for example, using physical models and optimization algorithms [2] or convolutional neural networks [3]. While these approaches have shown to improve image quality, a complete removal of motion artifacts remains challenging. This originates from the fact that the algorithms require image information that has never been acquired due to the occurred movement, generating gaps in the acquisition matrix. A general experience is, that these techniques only work well for smaller-scale, rigid bulk motion.

Prospective motion correction methods attempt to quantify the motion (e.g., translation, rotation) during the acquisition and adjust the scanner's coordinate system "live", following the patient's motion with the scanner's field-of-view. To this end, an accurate and fast estimation of the patient's position is required, using for instance cameras tracking a marker on the patient's face. This is, however, technically challenging, and further complicated by the fact that the tracking devices must operate in a very high magnetic field inside the MR bore, which makes these solutions also extremely expensive. Another way to accurately estimate motion for prospective motion correction are so-called "navigators". These are very fast scans which are interleaved with the actual imaging acquisition, informing on the patient's current position.

In particular, a technique called "pilot tone", which is similar to the above-mentioned navigator techniques has been recently developed for cardiac and respiration motion detection [4]. It uses a separate antenna to transmit a continuous radio-frequency (RF) wave signal, e.g. a sinusoidal RF signal, that is called "pilot tone signal" or simply "PT", which is transmitted close to the patients' body with a frequency close to the Larmor frequency of the magnetic resonance signal (but not the same). This pilot tone signal is modulated by occurring motion and can be recorded during the acquisition along with the actual MR image using the existing acquisition devices (MR coils and corresponding signal reception hardware). One advantage of this technique is its simplicity: it only needs a simple additional signal transmitted, which can be generated with simple extra hardware or even existing hardware depending on the scanner setup. The pilot tone signal is characterized by a frequency which can be picked up by the receiver hardware but is far enough from the frequency band used for the imaging process.

It has been shown that, in principle, motion of a biological object which is inside of an MRI scanner can be detected by the pilot tone through modulation of its amplitude and phase visible in the employed receive channels. However, the pilot tone signal is sensitive to various sources of noise and has intrinsic variations, both of which hamper the robust detection of motion. A robust pilot tone signal processing pipeline is therefore needed which provides reliable motion detection to manage the motion and improve the clinical workflow.

### Summary of Invention

An objective of the present invention is to propose a method and a system for robustly and automatically detecting a biological object motion during MRI that overcome the above-mentioned issues.

Said objective is achieved according to the present invention by a method and a system for automatically detecting a biological object motion during MRI according to the object of the independent claims. Dependent claims present further advantages of the invention.

The present invention concerns in particular a method for automatically detecting, during an MRI session, a motion of a biological object placed in an examination volume of an MRI apparatus, said examination volume being typically the bore of the MRI apparatus, the method comprising:
- emitting or generating, preferentially continuously or periodically, in said examination volume and during said MRI session, i.e. during a period of time during which said biological object is located in said examination volume, a pilot tone signal, hereafter "PT signal", wherein the PT signal is emitted at a frequency F_pt close to the Larmor frequency, but outside a range of frequencies used by the MRI apparatus for imaging said biological object, i.e. the frequency F_pt is outside the acquisition bandwidth, but inside the sampled frequency band with respect to the MRI signal induced in receiver coils of the MRI apparatus when imaging the biological object. During the MRI session, one or several scans (i.e. MRI pulse sequences for imaging the biological object) of the biological object might be performed or executed by the MRI apparatus. In other words, each MRI session according to the invention comprises an execution of one or several MRI pulse sequences, wherein two successive MRI pulse sequences can be separated by a time interval where the MRI apparatus pauses or performs certain preparatory steps for the following pulse sequence execution. During the execution of the MRI pulse sequence, i.e. the image acquisition, an MRI signal generated by the biological object is acquired by the MRI apparatus. Said emission or generation of the PT signal might take place for instance during or between the acquisition of MR signals of said biological object by means of said MRI apparatus. The frequency F_pt is preferentially comprised in a range of frequencies defined as the Larmor frequency ± 10% of its value. Typically, the PT signal is emitted or sent into the bore of the MRI apparatus, using for instance a transmitter or transmitting antenna for emitting said PT signal;
- during said MRI session, acquiring by the MRI apparatus and in real time the PT signal. The latter is typically received using the regular MRI signal receive pathway of the MRI apparatus. The amplitude of the PT signal is modulated by motion of the biological object in said examination volume. In particular, the PT signal is received by the same receiver coils used for acquiring the MRI signal, and preferentially by each of said receiver coils. Preferentially, the PT signal is emitted and acquired between two successive MRI pulse sequences (i.e. during said time interval) or during an MRI pulse sequence. In particular, the PT signal is received alongside the MRI signal used for imaging the biological object if the PT signal is emitted during acquisition of said MRI signal;
- detecting, during said MRI session, by a motion detection algorithm, in real time and from the acquired PT signal, a motion of the biological object;
- automatically triggering an action in function of the detected motion, said action being triggered during said MRI session, for instance while MRI acquisition is still ongoing for said biological object, notably during between or after the acquisition of an MR image.

The present invention also concerns a system for automatically detecting, during MRI session, a motion of a biological object placed in an examination volume of an MRI apparatus, the system comprising:
- a transmitter or transmitting antenna for emitting a pilot tone signal, hereafter PT signal, in said examination volume and during said MRI session;
- an MRI apparatus for acquiring the PT signal;
- a control unit comprising a processor, the control unit being configured for
   - detecting, by means of a motion detection algorithm, in real time and from the acquired PT signal, a motion of the biological object; and
   - automatically triggering an action in function of the detected motion,
said system being configured for carrying out the steps of the previously described method.

### Description of Embodiments

The present invention proposes to detect in real time a motion of the biological object from the PT signal that is acquired using existing MRI signal receiver hardware. The control unit uses said motion detection algorithm for detecting the motion. In addition, said motion detection algorithm might be configured for automatically calculating, for a detected motion, a quantifiable impact on image quality. In such a case, said action is preferentially automatically triggered only if said quantifiable impact exceeds a threshold, which can be predefined or dynamically determined in function of previously acquired PT signal data. In particular, the motion detection algorithm might comprise a machine learning algorithm configured for automatically calculating said quantifiable impact. Preferentially, said machine learning algorithm has been trained on a training dataset comprising for each reference biological object of a set of reference biological objects, one or several labelled images, and for each labelled image, an associated PT signal or a processed form of the PT signal, wherein the label of each labelled image provides a value of a quantifiable impact calculated for said labelled image. A reference biological object is notably a biological object of the same type as the one for which motion has to be detected, e.g. representing a same body portion.

Preferentially, said action according to the invention may comprise:
- alerting an operator, for instance by displaying a message and/or emitting an audible alert;
- automatically pausing or cancelling the ongoing acquisition;
- automatically launching a reacquisition of the image.

As mentioned above, said reacquisition is preferentially only launched if the quantifiable impact exceeds said threshold. In particular, the latter might be modified or tuned by an operator of the system according to the invention, in order to be adapted to said biological object.

Preferentially, the PT signal acquired by the receiver coils of the MRI apparatus is filtered before performing the detection of the motion of the biological object. In particular, the method according to the invention comprises calculating, for instance by means of said motion detection algorithm, a change of the amplitude of the PT signal in function of the time. For instance, it can be configured for calculating the derivative of the PT signal with respect to the time (temporal derivative). Said amplitude change in function of the time (also called temporal amplitude change) might then be used for detecting said motion by determining whether a change of the amplitude of the PT signal exceeded one of said thresholds during image acquisition.

In particular, the method step "acquiring the PT signal" comprises receiving by each RF receiver coil (or receiver channel) of the MRI apparatus said PT signal alongside the MRI signal. Then, the control unit of the system according to the invention is configured for processing the received PT signal in order to obtain a motion sensor signal that is used by the motion detection algorithm for detecting motion of the biological object. Said processing of the received PT signal might be implemented directly by the motion detection algorithm, or might involve additionally hardware processing and/or software processing. In particular, for all, or a predefined set, of the receiver channels, the control unit is configured for separating the received PT signal from the MRI signal (e.g. by using known in the art signal filtering) in order to obtain a separated PT signal for each receiver coil, i.e. for each receiver channel. Then, said control unit might be configured for combining all separated PT signals into a single PT signal, which can be said motion sensor signal used for the detection of motion. The filtering and/or said temporal amplitude change previously described may take place on each separated PT signal and/or after their combination into said single PT signal. For instance, a filtering might be applied to said single PT signal followed then by the calculation of its temporal amplitude change. The resulting PT signal, showing for instance temporal amplitude changes in function of the time, is then said motion detection signal used for detecting motions of the biological object. Alternatively, said filtering followed by the calculation of the temporal amplitude change might be performed for each separated PT signal, and only afterwards, the latter are combined into a single signal that is said motion sensor signal. At the end, the obtained motion sensor signal is used as input to said motion detection algorithm, and optionally displayed, for instance on a display of the MRI apparatus, so that an operator may follow the evolution of the motion sensor signal in real time. According to the present invention, the PT signal is received by all RF receiver coils, and thus by all RF receiver channels of the MRI apparatus, wherein each receiver coil comprises at least one electronic chain called receiver channel (typically comprising amplifiers, filters, analog-to-digital converter, demodulator, etc.) for processing the MRI signal and that is preferentially used for separating the received PT signal from the MRI signal.

According to the present invention, the PT signal might be emitted and acquired :
a. during said time interval between two consecutive scans, i.e. at a moment wherein no image acquisition takes place, i.e. at a moment wherein the signal received by the receiver coils is not used for image reconstruction; and/or
b. during a scan, i.e. during the MRI pulse sequence, being acquired in particular alongside the acquisition of the MRI signal used for reconstructing an image of the biological object.

Preferentially, the PT signal is emitted continuously during said MRI session and acquired between two consecutive scans and/or during a scan.

Preferentially, the method according to the invention may comprise applying, by the motion detection algorithm, one or several detection thresholds to said motion sensor signal for detecting said motion (i.e. timepoints when the biological object moved more than a predefined amount corresponding to said detection threshold).

### Brief Description of the Drawing

For a more complete understanding of the present disclosure, and the advantages thereof, reference is now made to the following descriptions taken in conjunction with the accompanying drawings, wherein like numbers designate like objects, and in which:
Fig. 1 illustrates a flowchart of a method for automatically detecting, during MRI, a motion of a biological object according to the invention;
Fig. 2 illustrates a preferred embodiment of a system according to the invention;
Fig. 3 shows a graph representing raw PT signal values in function ot the time;
Fig. 4 shows a graph representing the PT signal obtained after applying a median filter to the raw PT signal values of Fig. 3;
Fig. 5 shows a graph representing the PT signal obtained after applying a lowpass filter to the PT signal of Fig. 4;
Fig. 6 shows a graph representing scaled PT signals;

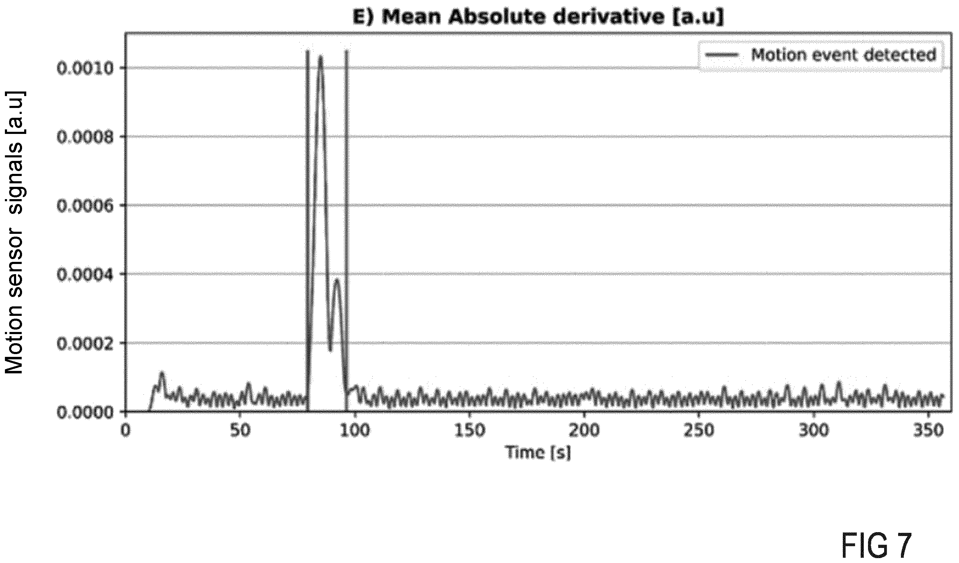

Fig. 7 shows a graph representing a mean absolute derivative of the scaled PT signals combined into a single PT signal.

### Description of Examples

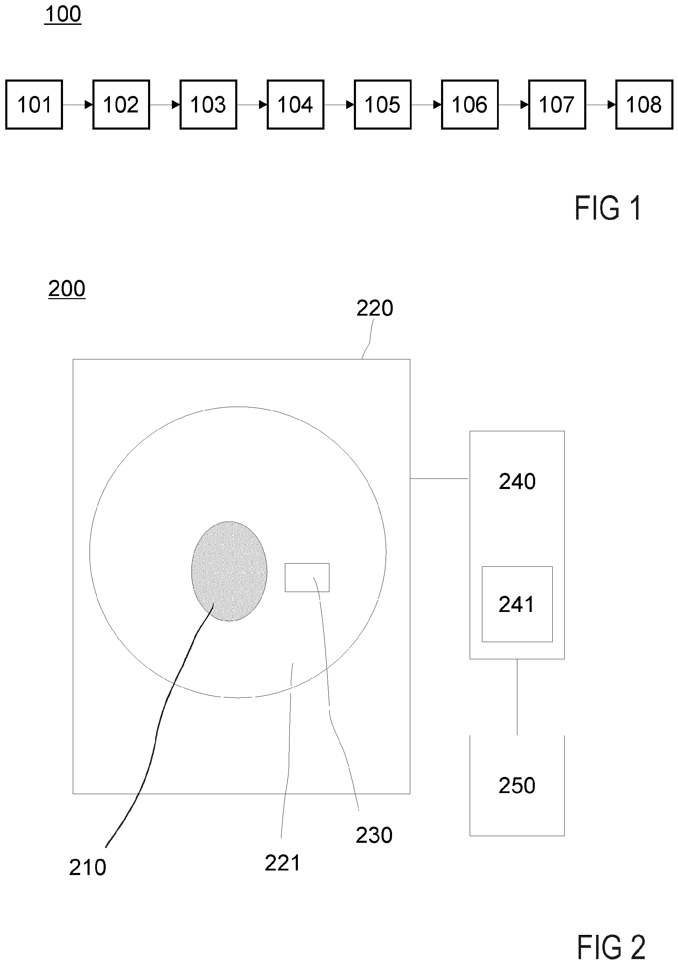

We will now describe in more detail a preferred embodiment of the method according to the invention through Figures 1 and 2, wherein Figure 1 describes the different steps of the method 100 carried out by a preferred embodiment of a system 200 according to the invention illustrated by Figure 2.

The present invention proposes to detect in real time motion of a biological object 210 during an MRI session, for instance during an MRI signal acquisition. In order to detect said motion, the present invention proposes to monitor changes of a PT signal via an MRI apparatus 220 during the MRI session, notably during MRI image acquisition. For this purpose, the system 200 comprises a transmitter 230 configured for emitting said PT signal in an examination volume 221 of the MRI apparatus 220. Changes observed in the PT signal received by receiver coils of the MRI apparatus 220 are an indication of a motion of the biological object 210, e.g. a patient's head or knee or shoulder or spine, in the examination volume 221, as the PT signal is modulated by changes of the electrical environment inside the examination volume 221, i.e. inside the receiver coils, which are induced by movement of biological tissue in said examination volume 221. The PT signal acquired by the MRI apparatus 220 is then processed by a control unit 240 that is configured for detecting motion from motion sensor signal obtained from the acquired PT signal. Said motion sensor signal is notably used as input to a motion detection algorithm that is configured for detecting motion of the biological object 210, and, optionally, for determining an impact of said motion on image quality. Preferentially, the motion sensor signal might be displayed in real time on a display 250 of the system 200.

Advantageously, the present invention enables to inform or notify an operator or another system, in real time during the MRI session about a detected motion of the biological object. For instance it enables to alert an operator or to notify another system during MRI acquisition (i.e. during the acquisition of an image by the MRI apparatus - in other words, while MRI data for said image are still under acquisition) about a detected motion of the biological object and optionally about its impact on the quality of the MRI image by quantifying or estimating a degradation of the image quality due to the detected motion. This makes the MRI acquisition far more efficient compared to existing techniques by enabling to trigger an appropriate action at a very early stage, for instance by stopping the current acquisition and starting a reacquisition of MRI data if a degradation of the image has been detected by the system according to the invention and exceeds for instance a "degradation" threshold, enabling thus to save time in the radiological workflow.

The different steps of the method illustrated in Fig. 1 will be described now in more details:
At step 101, during the period of time the biological object is placed in the examination volume 221, the transmitter 230 emits the PT signal. Preferentially, said PT signal is emitted during an acquisition of an image for said biological object 210. The transmitter 230 might be controlled by said control unit 240. The PT signal is emitting in the examination volume 221 wherein the biological object 210 is placed for the MRI data acquisition. The PT signal is preferentially continuously emitted during said period of time, i.e. throughout the MRI session or examination, i.e. before, during, and after the MRI data acquisition for imaging said biological object 210. Alternatively, the PT signal could be emitted only between MRI data acquisition, i.e. between two successive scans. The PT signal is emitted at a constant frequency, which is comprised within a range of radio frequencies capturable by the receiver coils of the MRI apparatus, but outside of a range of radio frequencies used by the MRI apparatus for imaging the biological object, so that the PT signal does not interfere with the MRI signal. Typically, the PT signal is a sine wave with constant amplitude and fixed frequency.

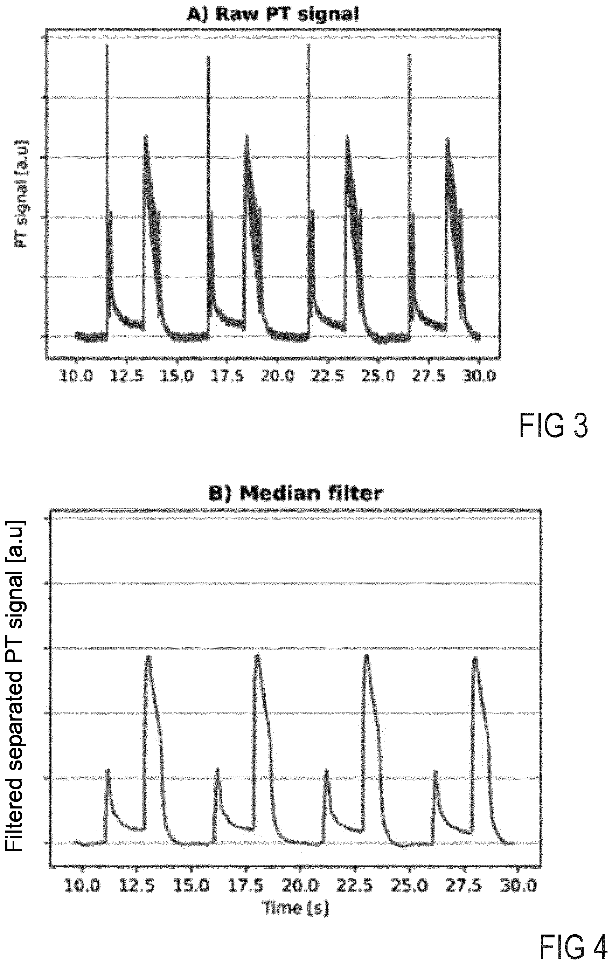

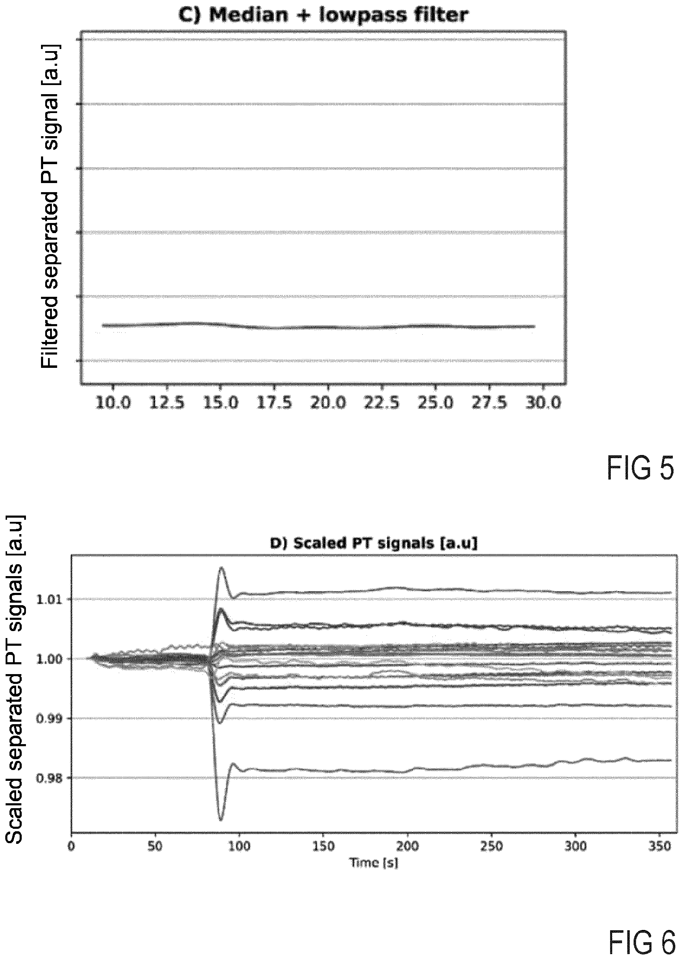

At step 102, the PT signal is acquired by the MRI apparatus 220, preferentially alongside the MRI signal. In particular, the emitted PT signal is received by all receiver coils, and thus all receiver channels of the MRI apparatus 220. Then the control unit 240 is configured for processing the signal received by each receiver coil. In a first step 102A, the signal received by each receiver coil is processed, e.g. using a filtering technique or any other known in the art signal processing based on software and/or hardware signal processing 241, for separating the PT signal from the MRI signal. This enables to collect, for each receiver coil a PT signal (called hereafter the "separated PT signal") separated from the MRI signal collected by said receiver coil and representing the raw PT signal received by a receiver coil. An example of such raw PT signal in function of the time is shown in Fig. 3. In a second step 102B, and preferentially, each separated PT signal might be further filtered to remove potential disturbances that are not related to motion of the biological object 210, but could originate from other MRI apparatus scanner hardware interference or signal drifts due to heating of hardware. Figure 4 presents an example of a separated PT signal obtained after application of a median filter on the raw PT signal of Figure 3. Preferentially, additional filtering techniques might be applied to each separated PT signal. For instance, the application of said median filter to each separated PT signal might be followed by two applications of Butterworth lowpass digital filters, resulting in the filtered separated PT signal illustrated in Fig. 5. In a third step 102C, a temporal derivative (i.e. the rate of change over time) of each separated PT signal is calculated by the control unit 240, for instance by the motion detection algorithm. Preferentially, their absolute derivative is calculated. Said temporal derivative provides a measurement of the variation of the, optionally filtered, separated PT signal in function of the time, representing thus how fast the PT signal has changed over time. In a fourth step 102D, all or a part of the, optionally filtered, separated PT signals are combined into a single PT signal, i.e. the so-called motion sensor signal. Said motion sensor signal might be obtained by averaging said all or part of the, optionally filtered, separated PT signals. Preferentially, before combining said separated PT signal, an additional scaling step takes place, wherein each, optionally filtered, separated PT signal is scaled independently to a mean value of one to mitigate inter-scan and inter-biological object differences. An example of scaled PT signals is presented in Fig. 6 where motion occurred at a time point of 85 s, wherein for each receiver coil, i.e. each of the separated PT signal has been scaled to a mean value of one. Figure 6 shows the motion sensor signal in function of the time obtained after the complete processing of the separated PT signals, computed by calculating the mean of the absolute derivative of the scaled and filtered separated PT signals. Said motion sensor signal might be displayed on a screen or display 250 in real time during MRI acquisition.

At step 103, the control unit 240 is configured for detecting, in real time and via its motion detection algorithm, from the obtained motion sensor signal, whether a motion of the biological object took place. For this purpose, the motion detection algorithm might be configured for automatically determining whether the motion sensor signal exceeds a threshold or not, wherein said threshold might be pre-defined or dynamically assessed from previously acquired PT signal data (e.g. from previously acquired motion sensor signal). In particular, if said threshold has been exceeded during an image acquisition, then the concerned image is labelled as affected by motion, while it is labelled as non-affected by motion if said threshold has not been exceeded. Said labelled images might be used for training a machine learning algorithm configured for predicting the degradation of image quality due to biological object motion.

At step 104, the control unit 240 is configured for automatically triggering an action while the biological object is still in the examination volume, notably while MRI acquisition is still ongoing, for instance during said acquisition of the image for the biological object. Said action is triggered in function of the detected motion, notably in function of an amplitude of the motion sensor signal. For instance, if said threshold is exceeded, then the motion detection algorithm might be configured for sending a message or data indicating that a relevant motion took place, and optionally, for stopping the current MRI data acquisition and restarting said MRI data acquisition at a stage directly preceding the time at which said relevant motion occurred.

Preferentially, in order to determine the image quality, additional thresholds can be applied to the motion sensor signal to detect different degrees of motion, i.e., timepoints when the biological object in the scanner of the MRI apparatus moved more than a predefined amount, and therefore different degrees of degradation of the image quality, wherein the operator may for instance chose a specific degree of motion in function of the biological object under examination. Preferentially, when a motion is detected, then its characteristics or features (e.g., timepoints during the MRI acquisition, sequence properties, multi-channel pilot tone signal amplitudes, amongst others) can be provided to said machine learning algorithm that is trained or configured to predict the image quality. The predicted image quality might then be communicated to the operator of the MRI apparatus. In particular, in order to determine or predict the image quality, i.e. for deciding if a motion will result in a moderate or severe image quality degradation, said machine learning algorithm might comprise a Support Vector Machine (SVM) which uses as input said characteristics or features extracted from a currently detected motion including for instance when the motion occurred and its amplitude, in order to determine a corresponding image quality, and thus also a corresponding degradation of the image quality. In order to train the machine learning algorithm, a training set comprising manually labelled images that have been previously acquired, and for each labelled image, its corresponding PT signal (or a signal obtained from the latter) or its corresponding motion sensor signal, that has been recorded for said labelled image, are provided as input to the machine learning algorithm, wherein the label of the labelled image is configured for providing a value of a quantifiable impact on image quality of motion that took place during acquisition of said labelled image. Typically, said value of the quantifiable impact might be determined for each labelled image of the training set by determining a loss of resolution in the acquired image compared to a reference image for which no motion occurred.

To conclude, the present invention proposes to use a PT signal to detect motion of a biological object during or between MRI acquisitions, predict the image quality before the MRI acquisition has finished, enabling to automatically trigger a relevant action like warning an operator of the MRI apparatus and/or implementing an automated workflow intervention, like restarting or repeating a scan. This can improve patient comfort and reduce the time required for performing MRI acquisition of images whose quality are higher than a threshold.

### List of citations

[1] M. Zaitsev, J. Maclaren, and M. Herbst, "Motion artifacts in MRI: A complex problem with many partial solutions," J. Magn. Reson. Imaging, vol. 42, no. 4, pp. 887-901, 2015
[2] A. Loktyushin, M. Babayeva, D. Gallichan, G. Krueger, K. Scheffler, and T. Kober, "Retrospective rigid motion correction of undersampled MRI data," in Intl. Soc. Mag. Reson. Med, 2015, program no. 2556.
[3] M. W. Haskell, S. F. Cauley, B. Bilgic, et al., "Network Accelerated Motion Estimation and Reduction (NAMER): Convolutional neural network guided retrospective motion correction using a separable motion model," Magn. Reson. Med., vol. 82, no. 4, pp. 1452-1461, 2019
[4] T. Vahle, M. Bacher, D. Rigie, et al., "Respiratory Motion Detection and Correction for MR Using the Pilot Tone: Applications for MR and Simultaneous PET/MR Examinations," Invest. Radiol., vol. 55, no. 3, pp. 153-159, 2020.

## Claims

1. Method (100) for automatically detecting, during an MRI session, a motion of a biological object (210) placed in an examination volume (221) of an MRI apparatus (220), the method (100) comprising:
- emitting (101), in said examination volume (221) and during said MRI session, a pilot tone signal - hereafter PT signal -, wherein the PT signal is emitted at a frequency F_pt close to the Larmor frequency, but outside a range of frequencies used by the MRI apparatus (220) for imaging said biological object (210);
- acquiring (102), during said MRI session and by the MRI apparatus (220), the PT signal;
- detecting (103), during said MRI session, by a motion detection algorithm, in real time and from the acquired PT signal, a motion of the biological object;
- automatically triggering (104) an action during said MRI session and in function of the detected motion.

2. Method (100) according to claim 1, wherein the motion detection algorithm is configured for automatically calculating, for a detected motion, a quantifiable impact on image quality, and said action is automatically triggered only if said quantifiable impact exceeds a threshold.

3. Method (100) according to claim 2, wherein the motion detection algorithm comprises a machine learning algorithm configured for automatically calculating said quantifiable impact, wherein said machine learning algorithm has been trained on a training dataset comprising for each reference biological object of a set of reference biological objects, one or several labelled images, and for each labelled image, an associated PT signal or a processed form of the PT signal, wherein the label of said labelled images provides a value of the quantifiable impact calculated for said labelled image.

4. Method (100) according to claim 2 or 3, wherein said threshold is predefined or dynamically determined in function of previously acquired PT signal data.

5. Method (100) according to one of the claims 1 to 4, wherein said action further comprises:
- alerting an operator;
- automatically pausing or cancelling the acquisition;
- automatically launching a reacquisition of the image.

6. Method (100) according to one of the claims 1 to 5, comprising filtering the acquired PT signal before performing said detection of a motion of the biological object.

7. Method (100) according to one of the claims 1 to 6, comprising calculating a change of the amplitude of the PT signal in function of the time.

8. Method (100) according to one of the claims 1-7, wherein acquiring the PT signal comprises receiving by each RF receiver channel of the MRI apparatus said PT signal alongside the MRI signal, then for all or a predefined set of the receiver channels, separating the received PT signal from the MRI signal in order to obtain a separated PT signal, combining all separated PT signals into a single motion sensor signal, and using said motion sensor signal as input to said motion detection algorithm.

9. Method (100) according to one of the claim 8, comprising applying, by the motion detection algorithm, one or several detection thresholds to said motion sensor signal for detecting said motion.

10. Method (100) according to claim 8 or 9, comprising displaying the motion sensor signal.

11. System (200) for automatically detecting, during an MRI session, a motion of a biological object (210) placed in an examination volume (221) of an MRI apparatus (220), the system (200) comprising:
- a transmitter (230) for emitting a pilot tone signal, hereafter PT signal, in said examination volume (221) and during said MRI session;
- said MRI apparatus (220) for acquiring the PT signal;
- a control unit (240) comprising a processor, the control unit (240) being configured for
- detecting, by means of a motion detection algorithm, in real time and from the acquired PT signal, a motion of the biological object (210); and
- automatically triggering an action in function of the detected motion,
said system (200) being configured for carrying out the steps of the method (100) according to one of the claims 1 to 10.
